# EUROPÄISCHE PATENTSCHRIFT

(11) **EP 0 682 499 B1**
(45) Veröffentlichungstag und Bekanntmachung des Hinweises auf die Patenterteilung: **13.08.1997**
(21) Anmeldenummer: 94906200.4
(22) Anmeldetag: 02.02.1994
(51) Int. Cl.: A61B 10/00

(54) **TESTPFLASTER ZUR EPICUTANEN HAUTTESTUNG**
TEST PATCH FOR EPICUTANEOUS SKIN TESTING
TIMBRE POUR TEST EPICUTANE

(30) Priorität: 06.02.1993 DE 4303495
(43) Veröffentlichungstag der Anmeldung: 22.11.1995
(73) Patentinhaber: Siewert, Ronald-R., c/o INNOV-ALL PHARMA VERTRIEBS GmbH & CO. KG, 40231 Düsseldorf (DE)
(72) Erfinder: SIEWERT, Ronald, R. I-A Ph.Vertr. GmbH & Co. KG, D-40231 Düsseldorf (DE); SCHMOLL, Manfred, D-26125 Oldenburg (DE)
(74) Vertreter: Meyers, Hans-Wilhelm, Dr.Dipl.-Chem.
(86) Internationale Anmeldenummer: EP9400293
(87) Internationale Veröffentlichungsnummer: WO9417735

(56) Entgegenhaltungen:
- WO-A-92/01421
- DE-C- 3 810 658
- US-A- 4 788 971

## Beschreibung

Gegenstand der vorliegenden Erfindung ist ein Testpflaster zur epicutanen Hauttestung gemäß Oberbegriff des Anspruchs 1.

Die DE-PS 24 20 345 beschreibt eine Hauttestdeckung zur Untersuchung der Wirkung eines Stoffes auf die menschliche Haut. Dabei befinden sich die zu testenden Substanzen in einem Abdeckungskörper, welcher einstückig aus einem Plattenteil und einem dessen Umfangsrand begrenzenden, mit der Haut in Berührung tretenden geraden Teil (Kragenteil) besteht. Der Abdeckungskörper wird mit einem Pflaster zur Befestigung auf der Haut versehen. Der Kragenteil ist dabei in Richtung der Hautoberfläche abgewinkelt und die zur abdichtenden Berührung mit der Haut dienende Kante ist abgerundet.

Das deutsche Gebrauchsmuster G 84 19 329 U1 betrifft ein Hauttestpflaster, das aus einer Befestigungsunterlage und einem oder mehreren daran befestigten Testzentren besteht. Die Testzentren sind an ihrer Unterseite an der mit einem Klebstoff überzogenen Unterlage befestigt.

Die WO 92/01421 betrifft ein Testpflaster mit quadratischen Testkammern, die auf einem Klebeband angeordnet sind und wobei die klebenden Stellen mit einer Schutzfolie abgedeckt sind. Die Schutzfolie weist Aussparungen an den Stellen auf, an denen die Testkammern auf der Klebefolie befestigt sind.

Die DE 38 10 658 betrifft ein Epicutan-Testpflaster, mit mindestens einer auf einer Trägerfolie angeordneten Wirkstoffaufnahmeeinrichtung, wobei die Trägerfolie ein für flüssiges Wasser dichtes, aber für Wasserdampf durchlässiges, hochelastisches Polymermaterial ist und an der der Haut abgewandten Fläche mit einer mindestens die Fläche der Trägerfolie abdeckenden Stützfolie ganzflächig oder an Teilflächenabschnitten lösbar verbunden ist. Die Wirkstoffaufnahmeeinrichtungen sind kreisrund ausgestaltet.

Bei sogenannten Epicutan-Testungen wird die Haut des Patienten über 24 bis 72 Stunden in Kontakt mit verschiedenen zu prüfenden Testsubstanzen gebracht, um die Reaktion der Haut und des Immunsystems auf diese Substanzen zu beobachten. Auf diese Weise soll festgestellt werden, ob bestimmte Substanzen als Allergene bei dem betreffenden Probanden wirken. Die zur Aufnahme der Allergene verwendeten Testkammern sind bei den im Stand der Technik verwendeten Testvorrichtungen aus Metall oder Kunststoffen gefertigt. Die Verwendung von metallischen Aufnahmereservoiren hat sich zunehmend als problematisch erwiesen, da Metallbestandteile der Testkammern mit metallischen Testallergenen in Wechselwirkung treten können. Die Verwendung rechteckiger Kammern hat zwar gegenüber der Verwendung kreisförmiger Kammern den Vorteil, daß der Arzt besser zwischen einer Störung der Hautfunktion und einer durch Testallergene hervorgerufenen Hautirritation unterscheiden kann. Rechteckige Kammern haben aber den Nachteil, daß die Ecken Hautreizungen hervorrufen können, die vom Arzt als falsch positive Hautreaktion gedeutet werden können und für Patienten mit erheblichen Schmerzen verbunden sein können.

Darüber hinaus hat sich die Verwendung der entsprechenden Pflasterfläche, die mit der Haut in Kontakt kommt, als problematisch erwiesen, da sie aufgrund des verwendeten, nichttransparenten Pflastermaterials keine permanenten Kontrolluntersuchungen erlauben und daher zur Beobachtung der Hautreaktion abgenommen werden müssen. Sie sind dann nur mit zusätzlichem Aufwand wieder am Patienten anzubringen. Desweiteren gewährleisten sie keinen hohen Tragekomfort. Dies ist für die Patienten, insbesondere bei längerer Testdauer, mitunter sehr unangenehm.

Aufgabe der vorliegenden Erfindung ist es somit, ein Testpflaster zur epicutanen Hauttestung bereitzustellen, das die genannten Nachteile des Standes der Technik vermeidet.

Die Aufgabe wird gelöst durch ein Testpflaster gemäß den Merkmalen des Anspruchs 1. Die sich daran anschließenden Unteransprüche betreffen bevorzugte Ausführungsformen des erfindungsgemäßen Testpflasters.

Die ovale Ausgestaltung der Kammer 10 ist vorteilhaft, da der Arzt zuverlässig zwischen einer unspezifischen, zufälligen, meist runden Hautveränderung und einer spezifischen, durch das Allergen hervorgerufenen Hautveränderung unterscheiden kann, die dann die ovale Form der Testkammer aufweist. Es ist mithin möglich, von anderen Hautirritationen, die sich punkt- bzw. kreisförmig ausbreiten, zu unterscheiden.

Figur 1 zeigt eine bevorzugte Ausführungsform des erfindungsgemäßen Testpflasters zur epicutanen Hauttestung von der Seite der Schutzfolie 30 her gesehen.

Figur 2 zeigt einen Schnitt durch das Testpflaster gemäß Figur 1 längs der Linie II ... II. Die Deckfolie 20 und Schutzfolie 30 überragt dabei die Trägerfolie 1 im Bereich 25.

Figur 3 zeigt die andere Seite des erfindungsgemäßen Testpflasters von der Seite der Deckfolie her gesehen. Durch die Vorstanzung 24 wird die Deckfolie 30 in einen äußeren Bereich 21 und einen davon getrennt entfernbaren mittleren Bereich 22 aufgeteilt.

Figur 4 zeigt eine perspektivische Darstellung der ovalen Kammer 10 zur Aufnahme der zu testenden Substanz. In der ovalen Kammer 10 ist ein saugfähiges Material, z. B. ein Vlies 15, angeordnet.

Figur 5 zeigt einen Querschnitt durch die ovale Kammer 10.

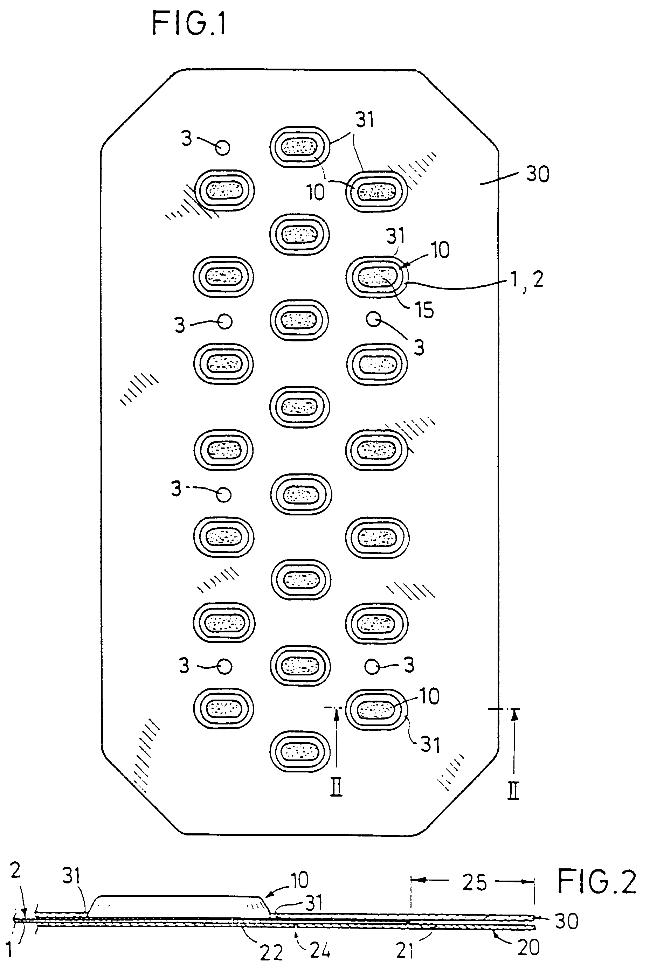

Das Testpflaster zur epicutanen Hauttestung gemäß der Erfindung wie es in Figur 1 dargestellt ist besteht aus einer Trägerfolie 1 und darauf angeordneten ovalen Kammern 10 zur Aufnahme der auf der Haut zu testenden Stoffe. Die Trägerfolie 1 ist dabei eine für Luft und Wasserdampf durchlässige, transparente Kunststoffolie, die auf einer Seite mit einem hautverträglichen Haftkleber 2 versehen ist. Auf dieser Seite der Trägerfolie 1 sind die ovalen Kammern 10 zur Aufnahme der zu testenden Stoffe angeordnet. Auf der mit Haftkleber 2 versehenen Seite gegenüberliegenden Seite der Trägerfolie 1 ist eine Deckfolie 20 angeordnet, wobei die Deckfolie 20 über die Trägerfolie 1 insbesondere auch umseitig hinausragt.

Diese Anordnung hat den Vorteil, daß aufgrund der Transparenz der Trägerfolie 1 die Beobachtung einer allergischen Reizung kontinuierlich beobachtet werden kann, ohne das Pflaster abziehen zu müssen und somit den Versuch in erheblichem Maße zu stören. Der Tragekomfort des erfindungsgemäßen Testpflasters wird durch die Luft- und Wasserdampfdurchlässigkeit der Trägerfolie 1 erheblich erhöht.

Durch die Verwendung einer steiferen Deckfolie 20 läßt sich die Handhabung des erfindungsgemäßen Testpflasters, insbesondere bei Verwendung einer sehr dünnen Trägerfolie 1 aus Kunststoff, erheblich erleichtern, dies besonders bei Befüllen der ovalen Kammern 10 mit den allergenen Substanzen.

In einer bevorzugten Ausführungsform des erfindungsgemäßen Testpflasters ist dieses an der mit Haftkleber 2 versehenen Seite der Trägerfolie 1 mit einer Schutzfolie 30 abgedeckt. Vorzugsweise sind in der Schutzfolie 30 Aussparungen 31 für die ovalen Kammern 10 vorgesehen. Die Anbringung einer Schutzfolie 30 erleichtert die Handhabung des erfindungsgemäßen Testpflasters dadurch, daß beim Befüllen und Applizieren des Testpflasters die klebenden Stellen der Trägerfolie 1 nicht an den Fingern haften bleiben und damit die Klebefähigkeit des Testpflasters erheblich reduziert wird.

Vorzugsweise ist die Schutzfolie 30 mit der Deckfolie 20 kongruent, d. h. die Folien 30 und 20 besitzen identische Ausdehnungen. Dabei ist die Trägerfolie 1 sandwichartig zwischen der Deckfolie 20 und der Schutzfolie 30 eingeschlossen. Da die Folien 20 und 30 jeweils selbst nicht klebend sind und jeweils die Trägerfolie 1 überragen, wird eine Entfernung der jeweiligen Folien 20 und 30 erleichtert.

Die Figur 2 zeigt einen Querschnitt durch das erfindungsgemäße Testpflaster gemäß Figur 1 längs der Linie II ... II.

Die Trägerfolie 1 des erfindungsgemäßen Testpflasters besteht vorzugsweise aus Polyurethan mit einer Stärke von 15 bis 35 µm. Vorzugsweise ist die Trägerfolie 1 hochelastisch und dehnbar bis 700 %. Als besonders bevorzugt zum Einsatz im erfindungsgemäßen Pflaster hat sich eine im klinischen Bereich seit Jahren bewährte Polyurethanfolie, die unter der Bezeichnung Tegaderm® der Firma 3M, Minnesota, USA erhältlich ist, herausgestellt.

Der auf der Klebeseite der Trägerfolie 1 des erfindungsgemäßen Testpflasters angeordnete Haftkleber 2 ist vorzugsweise ein hypoallergener Haftklebstoff auf Acrylatbasis, der aufgrund seiner Zusammensetzung gleichzeitig eine gute Haftfähigkeit z.B. auf fettiger Haut gewährleistet und nach Abschluß der Testphase ein Abziehen des Pflasters ohne Schmerzen ermöglicht. Der Acrylatkleber sollte zudem vorzugsweise nur geringe Wasserlöslichkeit aufweisen, um ein Ablösen des Heftpflasters von der Haut z.B. beim Schwitzen oder beim Baden, Duschen oder Saunen zu vermeiden. Das erfindungsgemäße Testpflaster ermöglicht aufgrund seiner zwar wasserdampfdurchlässigen aber wasserabweisenden Eigenschaften dem Patienten hin und wieder Aktivitäten wie Duschen, Schwimmen etc, ohne den Test erheblich einzuschränken.

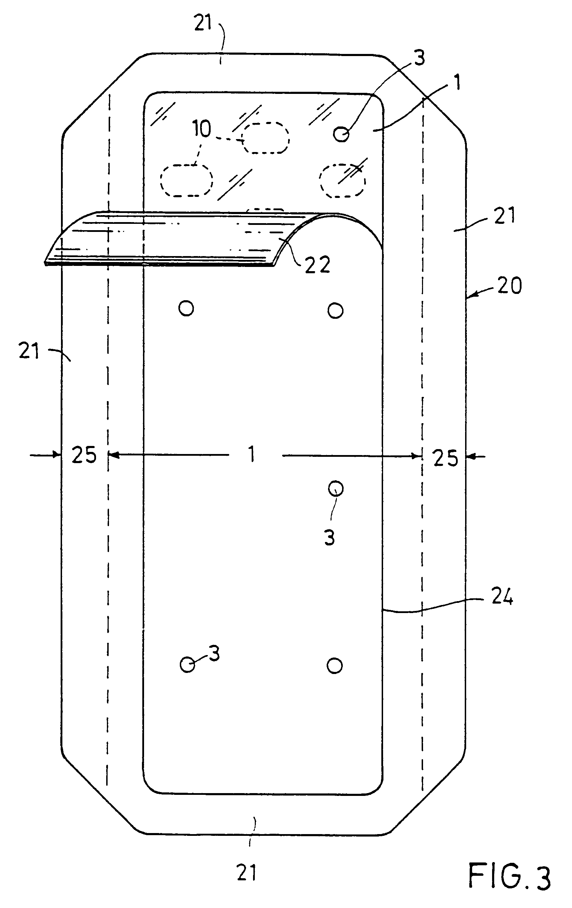

Die Figur 3 zeigt eine Aufsicht auf das erfindungsgemäße Pflaster, und zwar auf die Deckfolie 20. Vorzugsweise ist die Deckfolie 20 mehrteilig ausgestaltet. Die Deckfolie 20 des erfindungsgemäßen Testpflasters kann dabei durch Stanzung 24 in einem Bereich 21, der den Rand in einer gewissen Breite umfaßt, und dem mittleren Teil 22 getrennt sein. Nach dem Befüllen der ovalen Kammern 10 mit den Testsubstanzen wird zum Gebrauch die Schutzfolie 30 abgezogen. Der die Klebseite der Trägerfolie 1 überragende Teil 25 der Deckfolie 20 dient zum sicheren Fassen des Testpflasters. Danach kann der mittlere Teil 22 der Deckfolie 20 abgezogen werden, so daß der Rahmen 21 auf der nicht mit Haftkleber 2 versehenen Seite der Trägerfolie 1 verbleibt. Das durch das Abziehen des mittleren Teils 22 der Deckfolie 20 entstehende Fenster ermöglicht die Sicht auf die Kammern 10 zur exakten Positionierung des gesamten Pflasters. Nachdem das erfindungsgemäße Testpflaster mit der klebenden Seite der Trägerfolie 1 auf der Haut des Patienten angeordnet ist, kann der verbliebene Rest der Deckfolie 20, der Rahmen 21, von der Oberseite der Trägerfolie 1 abgezogen werden.

Durch die gegebenenfalls in der Trägerfolie 1 befindlichen vorgestanzten Löcher 3 kann eine Markierung des Testfeldes auf der Haut erfolgen.

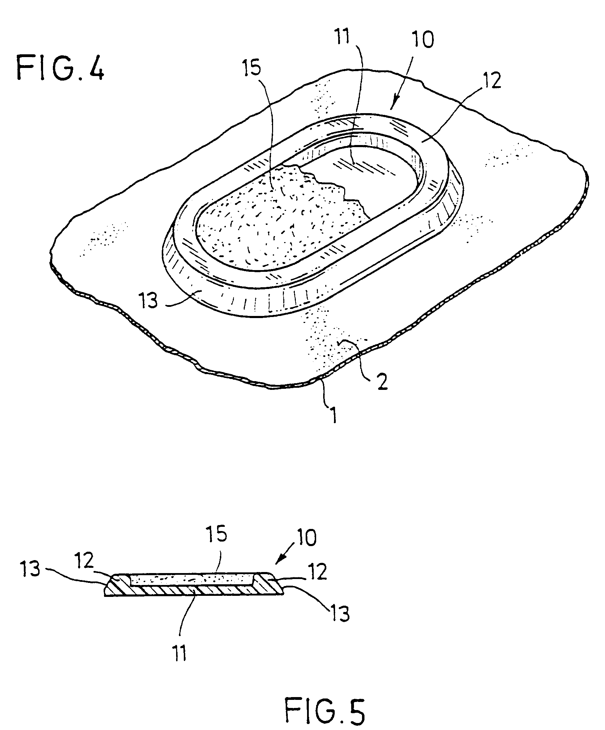

Die auf der klebenden Seite der Trägerfolie 1 angeordneten ovalen Kammern 10 sind in Figur 4 näher beschrieben. Vorzugsweise werden Testkammern aus hautverträglichem Kunststoff wie Polyethylen eingesetzt.

In einer besonders bevorzugten Ausführungsform ist die ovale Kammer 10 aus einem transparenten Material gefertigt. Die transparente ovale Kammer 10 in Verbindung mit der transparenten Trägerfolie 1 hat den Vorteil, daß eine permanente Beobachtung der unter der ovalen Kammer 10 befindlichen Haut, die den in der Kammer befindlichen Allergenen ausgesetzt ist, gewährleistet ist.

Die Figur 5 zeigt den Querschnitt durch eine ovale Kammer 10 des erfindungsgemäßen Testpflasters. Die ovale Kammer 10 ist vorzugsweise einstückig ausgebildet, wobei das innere Lumen der Testkammer durch einen Boden 11 und rings umherlaufender Wandung 12 zur Erzielung eines okklusiven Verschlusses zwischen Testkammer und Hautareal gebildet wird. Die innere Seite der Wandung 12 bildet mit dem Boden 11 vorzugsweise einen rechten Winkel. Die obere Begrenzung der Wandung 12 ist parallel zur Bodenfläche ausgebildet. Die äußere Wandung 13 fällt vorzugsweise nicht rechtwinklig zur Bodenplatte hin ab, sondern weist vorzugsweise einen Böschungswinkel (Winkel zwischen Boden 11 und äußerer Wandung 12) < 90° auf. Eine besonders bevorzugte Gestaltung der Wandung der ovalen Kammern 10 ist im Schnittbild der Figur 5 gezeigt.

Die ovale Kammer 10 weist vorzugsweise eine Innenfläche von 50 bis 70 mm² auf. Die Abstände zwischen den einzelnen Testkammern sind so groß gewählt, daß es nicht zu einander beeinflussenden Reaktionen der Testallergene auf der Haut kommt.

Im inneren Lumen der ovalen Kammern 10 befindet sich ein Vlies 15 oder eine andere saugfähige Unterlage fest angeordnet, um das Auslaufen der Testflüssigkeit aus den ovalen Kammern 10 zu verhindern. Besonders bevorzugt ist die Verwendung von transparenten Vliesen, die im Zusammenspiel mit der Transparenz der Kammern und der Trägerfolie ebenfalls eine dauernde Beobachtung der mit Allergenen kontaktierten Stelle erlauben.

Vorzugsweise sind auf der klebenden Seite der Trägerfolie 1 zweiundzwanzig ovalen Kammern 10 in drei Reihen gemäß Figur 1 angeordnet. Die Anordnung von 22 Testkammern ist vorteilhaft, da dies der europäischen Standardreihe zur Hauttestung von Allergenen entspricht. Vorteilhaft an den ovalen Kammern 10 des erfindungsgemäßen Testpflasters ist die Tatsache, daß deren vorzugsweise ovale Form eine bessere Unterscheidung allergischer Reaktionen von anderen Störungen der Hautfunktion ermöglicht. Die ovalen Kammern 10 weisen keine scharfen Winkel auf, welche Abdrücke auf der Haut hinterlassen und den Patienten beim Liegen Schmerzen bereiten könnten. Durch die Verwendung von Polyethylen zur Herstellung der ovalen Kammern 10 werden Interaktionen und Unverträglichkeiten bei Metalltestungen zwischen dem Kammermaterial und den Testallergenen vermieden, die auftreten können, wenn Kammern aus Metall verwendet werden.

Die ovalen Kammern 10 werden vorzugsweise mit ihrer Bodenplatte 11 direkt auf der klebenden Seite der Trägerfolie 1 angeordnet. Dabei ist es vorteilhaft die Unterseite der Kammern, die mit dem Kleber der Trägerschicht 1 in Berührung kommen, vorher durch Coronaentladung oder chemische Verfahren zu aktivieren. Dies führt zu einer intensiven Haftung der ovalen Kammern 10 auf der klebenden Seite der Trägerfolie 1.

Die Schutzfolie 30 bedeckt vorzugsweise die nicht von den ovalen Kammern 10 bedeckten klebenden Stellen der Trägerfolie 1 ab. Dazu ist es vorteilhaft, die Schutzfolie 30 mit Löchern 31 zu versehen, die möglichst genau dem Umfang und der Position der ovalen Kammern 10 auf der Trägerfolie 1 entsprechen.

Auf der Schutzfolie 30 können Informationen zur Verwendung des erfindungsgemäßen Pflasters aufgedruckt sein.

Die Handhabung des Testpflasters wird im folgenden näher beschrieben. Das erfindungsgemäße Testpflaster wird zur Vorbereitung der epicutanen Hauttestungen so plaziert, daß die Schutzfolie 30 nach oben zu liegen kommt. Danach werden die einzelnen Testallergene in die ovalen Kammern 10 gegeben. Es empfiehlt sich dabei, eine bestimmte Reihenfolge der Füllung einzuhalten. Beispielsweise kann die Beschickung mit der rechts oben liegenden ovalen Kammer 10 beginnen. Es empfiehlt sich, die ovalen Kammern 10 von oben nach unten zu befüllen.

Danach kann die Schutzfolie abgezogen werden. Dabei kommt die Trägerfolie 1 insgesamt zum Vorschein. Da die Deckfolie 20 jedoch die Testfolie 1 überragt, bleibt dadurch praktisch ein nicht klebender Bereich übrig, welcher die klebende Seite der Trägerfolie 1 in Form eines Rahmens umgibt. An diesem Rahmen kann das Testpflaster gehalten werden, ohne die klebenden Bereiche zu berühren. Nachdem die Testallergene in die ovalen Kammern 10 abgefüllt wurden und gegebenenfalls von dem in den ovalen Kammern 10 angeordneten Vlies aufgesaugt worden sind, kann das Testpflaster umgedreht werden und der hintere Bereich 22 der vorgestanzten Deckfolie 20 abgezogen werden. Es kommen dann die Unterseiten der ovalen Kammern 10 zum Vorschein. Danach wird das Pflaster mit der klebenden Seite auf die Haut des Patienten aufgebracht. Dabei sollte das Testpflaster auf die vorgespannte Haut, zum Beispiel dem gekrümmten Rücken, aufgetragen werden. Durch diese Maßnahme wird die Haftfähigkeit des Pflasters gesteigert und der Patient erhält einen größeren Bewegungsspielraum. Wenn das Testpflaster auf der Haut fixiert ist, wird der äußere Bereich 21 der Deckfolie ebenfalls abgezogen. Gegebenenfalls kann durch die in der Trägerfolie angebrachten gestanzten Markierungslöcher 3 eine Markierung auf der Haut entsprechend einer vorgefertigten Schablone angebracht werden.

Das erfindungsgemäße Testpflaster läßt sich durch einfaches Zerschneiden in kleinere Formate umwandeln, sofern die 22 ovalen Kammern nicht alle benötigt werden.

Das erfindungsgemäße Testpflaster kann einzeln oder in Gruppen verpackt vertrieben werden. Grundsätzlich ist eine Sterilverpackung nicht erforderlich.

## Patentansprüche

1. Testpflaster zur epicutanen Hauttestung mit einer Trägerfolie (1) und darauf angeordneten Kammern (10) zur Aufnahme der auf der Haut zu testenden Stoffe, wobei
- die Trägerfolie (1) eine für Luft und Wasserdampf durchlässige, transparente Kunststofffolie ist, die auf einer Seite mit einem hautverträglichen Haftkleber (2) versehen ist,
- auf dieser Seite die Kammern mit ovaler Form (10) zur Aufnahme der zu testenden Stoffe angeordnet sind,
- auf der gegenüberliegenden Seite die Trägerfolie (1) mit einer Deckfolie (20) abgedeckt ist, wobei die Deckfolie (20) über die Trägerfolie (1) hinausragt, dadurch gekennzeichnet, daß die Deckfolie (20) und/oder die Trägerfolie (1) mit vorgestanzten Markierungslöchern (3) versehen ist.

2. Testpflaster nach Anspruch 1, dadurch gekennzeichnet, daß die mit Haftkleber (2) versehene Seite der Trägerfolie (1) mit einer Schutzfolie (30) abgedeckt ist.

3. Testpflaster nach Anspruch 2, dadurch gekennzeichnet, daß die Schutzfolie (30) eine mit der Deckfolie (20) identische Ausdehnung besitzt und kongruent mit der Deckfolie (20) angeordnet ist.

4. Testpflaster nach mindestens einem der Ansprüche 1 bis 3, dadurch gekennzeichnet, daß die Trägerfolie (1) aus Polyurethan besteht.

5. Testpflaster nach mindestens einem der Ansprüche 1 bis 4, dadurch gekennzeichnet, daß die ovalen Kammern (10) aus einem transparenten Material wie Polyethylen bestehen.

6. Testpflaster nach mindestens einem der Ansprüche 1 bis 5, dadurch gekennzeichnet, daß die Deckfolie (20) mehrteilig ist.

7. Testpflaster nach Anspruch 6, dadurch gekennzeichnet, daß die Deckfolie (20) durch Stanzung in einen Rahmen (21) und einen getrennt davon abziehbaren mittleren Teil (22) getrennt ist.

8. Testpflaster nach mindestens einem der Ansprüche 1 bis 7, dadurch gekennzeichnet, daß die Polyurethan-Trägerfolie eine Stärke von 15 bis 35 µm aufweist.

9. Testpflaster nach mindestens einem der Ansprüche 1 bis 8, dadurch gekennzeichnet, daß der hautverträgliche Haftkleber (2) ein hypoallergener Acrylathaftkleber ist.

10. Testpflaster nach mindestens einem der Ansprüche 1 bis 9, dadurch gekennzeichnet, daß die Kammern (10) eine Innenfläche von 50 bis 70 mm² aufweisen.

11. Testpflaster nach mindestens einem der Ansprüche 1 bis 10, dadurch gekennzeichnet, daß die ovalen Kammern (10) ein saugfähiges, insbesondere transparentes Vlies zur Aufnahme der zu testenden Substanzen enthalten.

12. Testpflaster nach mindestens einem der Ansprüche 1 bis 11, dadurch gekennzeichnet, daß die Kammern (10) in mehreren parallelen Reihen auf der Trägerfolie (1)angeordnet sind.

13. Testpflaster nach mindestens einem der Ansprüche 1 bis 12, dadurch gekennzeichnet, daß die Schutzfolie (30) Aussparungen (31), die den Kammerpositionen auf der Trägerfolie (1) entsprechen, aufweist.

14. Testpflaster nach mindestens einem der Ansprüche 1 bis 13, dadurch gekennzeichnet, daß die Abstände zwischen den einzelnen Testkammern so groß gewählt wird, daß es nicht zu einander beeinflussenden Reaktionen der Testallergene auf der Haut kommt.

## Claims

1. A test plaster for epicutaneous skin testing having a support sheet (1) and compartments (10) arranged thereon for receiving the substances to be tested on the skin, wherein
- said support sheet (1) is a transparent plastic sheet which is permeable to air and water vapor and which is provided with a skin-compatible pressure-sensitive adhesive (2) on one side thereof;
- the compartments having an oval shape (10) for receiving the substances to be tested are arranged on said one side thereof; and
- said support sheet (1) is covered by a cover sheet (20) on the opposite side wherein said cover sheet (20) projects beyond said support sheet (1),
characterized in that said cover sheet (20) and/or said support sheet (1) are provided with pre-punched marking holes (3).

2. The test plaster according to claim 1, characterized in that the side of said support sheet (1) provided with pressure-sensitive adhesive (2) is covered by a protective sheet (30).

3. The test plaster according to claim 2, characterized in that said protective sheet (30) is identical in dimension with said cover sheet (20) and is arranged in a superposed condition with said cover sheet (20).

4. The test plaster according to at least one of claims 1 to 3, characterized in that said support sheet (1) is made of polyurethane.

5. The test plaster according to at least one of claims 1 to 4, characterized in that said oval compartments (10) are made of a transparent material, such as polyethylene.

6. The test plaster according to at least one of claims 1 to 5, characterized in that said cover sheet (20) consists of several parts.

7. The test plaster according to claim 6, characterized in that said cover sheet (20) is separated by punching into a frame (21) and a middle part (22) which can be separately released.

8. The test plaster according to at least one of claims 1 to 7, characterized in that the polyurethane support sheet has a thickness of from 15 to 35 µm.

9. The test plaster according to at least one of claims 1 to 8, characterized in that said skin-compatible pressure-sensitive adhesive (2) is a hypoallergenic acrylate pressure-sensitive adhesive.

10. The test plaster according to at least one of claims 1 to 9, characterized in that said compartments (10) have an interior surface area of from 50 to 70 mm².

11. The test plaster according to at least one of claims 1 to 10, characterized in that said oval compartments (10) contain an absorbent, especially transparent non-woven material for receiving the substances to be tested.

12. The test plaster according to at least one of claims 1 to 11, characterized in that said compartments (10) are arranged on said support sheet (1) in a number of parallel rows.

13. The test plaster according to at least one of claims 1 to 12, characterized in that said protective sheet (30) has recesses (31) corresponding to the positions of said compartments on said support sheet (1).

14. The test plaster according to at least one of claims 1 to 13, characterized in that the distances between the individual test compartments are chosen so large that no mutually interfering reactions of the test allergens will occur on the skin.

## Revendications

1. Emplâtre d'épreuve pour faire un test de la peau épicutané, comprenant une feuille de support (1) et des compartiments (10) disposés là-dessus pour recevoir les substances à tester sur la peau,
- ladite feuille de support (1) étant une feuille plastique transparente perméable à l'air et à la vapeur d'eau pourvue d'une composition autoadhésive (2) compatible avec la peau sur une de ses cotés;
- les compartiments en forme ovale (10) pour recevoir les substances à tester étant disposés sur cette coté; et
- ladite feuille de support (1) étant recouverte d'une feuille de recouvrement (20) sur la coté opposée, ladite feuille de recouvrement (20) dépassant ladite feuille de support (1),
caractérisé en ce que ladite feuille de recouvrement (20) et/ou ladite feuille de support (1) sont pourvues de trous de marquage (3) poinçonnés en avance.

2. Emplâtre d'épreuve selon la revendication 1, caractérisé en ce que ladite coté de la feuille de support (1) pourvue d'une composition autoadhésive (2) est recouverte d'une feuille protectrice (30).

3. Emplâtre d'épreuve selon la revendication 2, caractérisé en ce que ladite feuille protectrice (30) est étendue d'une manière identique à celle de ladite feuille de recouvrement (20) et est arrangée coïncidamment avec ladite feuille de recouvrement (20).

4. Emplâtre d'épreuve selon au moins une des revendications 1 à 3, caractérisé en ce que ladite feuille de support (1) consiste en du polyuréthane.

5. Emplâtre d'épreuve selon au moins une des revendications 1 à 4, caractérisé en ce que lesdites compartiments ovales (10) consistent en un matériau transparent, tel que le polyéthylène.

6. Emplâtre d'épreuve selon au moins une des revendications 1 à 5, caractérisé en ce que ladite feuille de recouvrement (20) consiste en plusieurs parties.

7. Emplâtre d'épreuve selon la revendication 6, caractérisé en ce que ladite feuille de recouvrement (20) est séparée par poinçonnement en un cadre (21) et une partie médiane (22) qui peut être détachée séparément.

8. Emplâtre d'épreuve selon au moins une des revendications 1 à 7, caractérisé en ce que ladite feuille de support en polyuréthane a un épaisseur comprise entre 15 et 35 µm.

9. Emplâtre d'épreuve selon au moins une des revendications 1 à 8, caractérisé en ce que ladite composition autoadhésive (2) compatible avec la peau est une composition autoadhésive hypoallergène sur base d'acrylate.

10. Emplâtre d'épreuve selon au moins une des revendications 1 à 9, caractérisé en ce que lesdites compartiments (10) ont une surface intérieure comprise entre 50 et 70 mm².

11. Emplâtre d'épreuve selon au moins une des revendications 1 à 10, caractérisé en ce que lesdites compartiments ovales (10) contiennent un toison absorbant et notamment transparent pour recevoir les substances à tester.

12. Emplâtre d'épreuve selon au moins une des revendications 1 à 11, caractérisé en ce que lesdites compartiments (10) sont arrangés sur ladite feuille de support (1) en plusieurs rangées parallèles.

13. Emplâtre d'épreuve selon au moins une des revendications 1 à 12, caractérisé en ce que ladite feuille protectrice (30) présente des évidements (31) correspondant aux positions des compartiments sur ladite feuille de support (1).

14. Emplâtre d'épreuve selon au moins une des revendications 1 à 13, caractérisé en ce que les distances entre les compartiments de test sont choisies si grands qu'il n'y a pas de réactions d'influence réciproques entre les allergènes de test sur la peau.
